# FASCICULE DE BREVET EUROPEEN

(11) **EP 4 059 565 B1**
(45) Date de publication et mention de la délivrance du brevet: **23.11.2022**
(21) Numéro de dépôt: 21315036.0
(22) Date de dépôt: 15.03.2021
(51) Int. Cl.: A61N 1/375, A61B 5/00, A61N 1/372, A61N 1/05

(54) **CAPSULE CARDIAQUE AUTONOME IMPLANTABLE ET EXTRACTIBLE À TÊTE ORIENTABLE ET LIMITEUR DE COUPLE**
AUTONOM IMPLANTIERBARE UND EXTRAHIERBARE HERZKAPSEL MIT SCHWENKKOPF UND DREHMOMENTBEGRENZER
IMPLANTABLE AND REMOVABLE AUTONOMOUS CARDIAC CAPSULE WITH ADJUSTABLE HEAD AND TORQUE LIMITER

(43) Date de publication de la demande: 21.09.2022
(73) Titulaire: Cairdac, 92160 Antony (FR)
(72) Inventeur: Regnier, Willy, 91160 Longjumeau (FR); Nguyen-Dinh, An, 37520 La Riche (FR)
(74) Mandataire: Dupuis-Latour, Dominique

(56) Documents cités:
- US-A1- 2014 378 991
- US-A1- 2014 378 992
- US-A1- 2020 094 048

## Description

### Domaine de l'invention

L'invention concerne les dispositifs médicaux implantables, notamment les dispositifs de type capsule autonome implantable.

L'invention concerne plus particulièrement ceux de ces dispositifs qui se présentent sous forme d'une capsule autonome implantée dans une cavité cardiaque (ventricule, oreillette ou même cavité cardiaque gauche artérielle), ci-après désignée "capsule autonome", "capsule *leadless*" ou simplement "capsule" (le caractère autonome de la capsule n'étant pas en soi une caractéristique nécessaire de l'invention). Ces capsules autonomes sont dépourvues de toute liaison physique à un dispositif principal implanté (tel qu'un boitier de générateur d'impulsions de stimulation) ou non implanté (périphérique externe tel que programmateur ou dispositif de monitoring pour le suivi à distance du patient), et sont dénommées pour cette raison "*leadless*", pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

L'invention n'est toutefois pas limitée à un type particulier de capsule, ni même d'implant *leadless*, et elle est applicable indifféremment à de nombreux autres types de dispositifs médicaux implantables, quelle que soit leur destination fonctionnelle, cardiaque ou autre, par exemple à des capsules destinées à diffuser *in situ* un agent pharmacologique actif. Dans ce cas d'une application cardiaque, la capsule surveille en continu le rythme du patient et délivre si nécessaire au cœur des impulsions électriques de stimulation, de resynchronisation et/ou de défibrillation en cas de troubles du rythme détecté par la capsule, La capsule peut être une capsule épicardique, fixée à la paroi extérieure du cœur, ou bien une capsule endocavitaire, fixée à la paroi intérieure d'une cavité ventriculaire ou auriculaire, ou bien encore une capsule fixée sur une paroi d'un vaisseau à proximité du myocarde.

La capsule comprend divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Les US 2009/0171408 A1 (Solem), US 2015/374976 A1 (Regnier), US 2017/0151429 A1 (Regnier) et WO 2018/122244 A1 (Regnier) décrivent divers exemples de telles capsules *leadless* intracardiaques.

### Description de la technique antérieure

L'invention concerne plus particulièrement les problèmes liés à l'implantation *in situ* et l'explantation de telles capsules lorsque celles-ci sont pourvues à leur extrémité distale d'un organe d'ancrage apte à pénétrer dans les tissus d'une paroi corporelle au site d'implantation choisi.

Un exemple typique d'un tel organe d'ancrage comprend une vis hélicoïdale saillante prolongeant axialement le corps de la capsule et destinée à pénétrer dans le tissu cardiaque par vissage au site d'implantation.

Dans le cas des capsules endocavitaires (c'est-à-dire des capsules à fixer à la paroi intérieure d'une cavité ventriculaire ou auriculaire, par opposition aux capsules épicardiques, fixées à la paroi extérieure du cœur), la "délivrance", c'est-à-dire à la mise en place au site d'implantation, consiste à monter la capsule à l'extrémité du cathéter-guide d'un accessoire d'implantation, puis à la faire progresser le long du réseau veineux périphérique et la diriger jusqu'au site choisi, par exemple l'apex de la cavité ventriculaire droite. Une fois le site d'implantation atteint, le praticien imprime à la capsule par l'intermédiaire du cathéter-guide des mouvements combinés de translation axiale (pour faire progresser la capsule vers l'avant puis exercer une pression contre la paroi cardiaque) et de rotation de la capsule sur elle-même (pour opérer le vissage de l'organe d'ancrage dans l'épaisseur de la paroi cardiaque). Une fois la capsule fermement ancrée dans la paroi cardiaque, l'opérateur procède au "largage" de la capsule, c'est-à-dire à sa désolidarisation d'avec l'accessoire d'implantation, la capsule devenant alors totalement autonome.

Pour éviter, au stade de la fixation de la capsule dans la paroi, tout risque de carottage des tissus du fait d'un vissage excessif, il est impératif au moment du vissage de l'organe d'ancrage de ne pas dépasser un couple limite (ci-après "couple de carottage") au-delà duquel la vis d'ancrage risquerait de déchirer localement les tissus sous l'effet de la rotation de la vis sans avance de celle-ci, jusqu'à provoquer une lacération des tissus et, à l'extrême, une perforation de la paroi avec risque de tamponnade (en particulier dans le cas d'une implantation dans une paroi mince telle que le septum interauriculaire ou la zone apicale du ventricule droit).

Le US 2020/094048 A1 (Regnier) propose une solution mettant en œuvre un système de limitation de couple intrinsèque à la capsule, plus précisément un système comprenant une tête solidaire de la vis d'ancrage et mobile en rotation par rapport au corps de la capsule. Entre le corps de la capsule et la tête mobile est disposé un mécanisme de couplage à friction débrayable permettant d'empêcher la rotation de la tête mobile par rapport au corps de la capsule tant que le couple de réaction exercé par la vis d'ancrage est inférieur à un seuil prédéterminé, et d'autoriser cette rotation dès que le couple de réaction dépasse le seuil prédéterminé. Ce système de débrayage de la tête mobile, et donc de la vis d'ancrage, d'avec le corps de la capsule entraînée par le cathéter-guide permet de préserver les tissus au site d'implantation en évitant toute lacération ou carottage, et donc d'assurer une implantation de la capsule dans les meilleures conditions.

La présente invention s'intéresse à une autre étape de la manipulation de la capsule, qui est celle de l'explantation, c'est-à-dire du retrait de la capsule du site d'implantation, en la détachant de la paroi à laquelle elle avait été ancrée.

Il s'agit alors de dévisser la vis d'ancrage en imprimant au corps de la capsule un mouvement de rotation dans le sens du dévissage (c'est-à-dire dans le sens contraire des aiguilles d'une montre), inverse de celui qui avait été exercé pour l'implantation (dans le sens du vissage, c'est-à-dire dans le sens des aiguilles d'une montre). Le couple de dévissage est appliqué au corps de la capsule par un cathéter qui a été amené jusqu'à la cavité où est implantée la capsule ; la partie distale du cathéter comprend un moyen permettant de saisir la partie arrière de la capsule (partie proximale, c'est-à-dire celle opposée à la paroi cardiaque) afin de se coupler à celle-ci et de pouvoir entraîner en rotation le corps de la capsule et, par voie de conséquence, l'ensemble frontal et la vis d'ancrage.

Si la capsule vient d'être récemment posée, ou si l'explantation consiste simplement, au cours de l'implantation, à retirer la capsule d'un premier site pour tenter d'en rechercher un meilleur, cette procédure ne pose pas de difficulté particulière.

En revanche, les études cliniques ont montré que, notamment après une longue période d'implantation, une fibrose se développe au niveau du site d'implantation autour de la vis d'ancrage ; cette dernière peut également se trouver plus ou moins prise dans les trabéculations du fond du ventricule lorsqu'un tel site est choisi.

Il faut alors que le couple de friction entre les deux éléments conjugués du mécanisme de couplage (c'est-à-dire entre l'ensemble frontal mobile et le corps de la capsule) soit suffisant pour transmettre à la vis le couple de dévissage exercé depuis l'arrière du corps de la capsule. En particulier, si le site est fibrosé, le corps de capsule risque de tourner dans le vide avec la vis immobile en place.

Dans le système décrit par le US 2020/094048 A1 précité, le mécanisme de couplage débrayable avec limiteur de couple permet de gérer la transmission du couple sur le muscle cardiaque lors du vissage de la capsule au moment de l'implantation. Mais le couple de débrayage s'appliquant de façon indifférenciée dans les deux sens de rotation (vissage et dévissage), à l'explantation le mécanisme de couplage à friction risque de se trouver en permanence dans un état débrayé du fait du couple de réaction élevé exercé par l'adhérence de la vis d'ancrage à la paroi cardiaque.

Le but de la présente invention est de résoudre ce problème d'une friction du mécanisme de couplage débrayable insuffisante pour transmettre un couple de dévissage lors d'une opération d'explantation d'une capsule déjà implantée.

### RÉSUMÉ DE L'INVENTION

L'idée de base de l'invention consiste à prévoir un mécanisme de couplage débrayable à friction s'appliquant de façon différenciée dans les deux sens de rotation (vissage/dévissage), de manière à annuler l'effet du débrayage uniquement dans le sens du dévissage, sans modifier le fonctionnement du couplage débrayable dans le sens du vissage.

À cet effet, l'invention propose une capsule autonome implantable du type décrit par le US 2020/094048 A1 précité, correspondant au préambule de la revendication indépendante 1.

Pour résoudre le problème précité, cette capsule comprend, selon les enseignements de la présente invention, les éléments énoncés dans la partie caractérisante de la revendication 1.

Les sous-revendications visent des modes de réalisation subsidiaires, avantageux de l'invention.

### DESCRIPTION SOMMAIRE DES DESSINS

On va maintenant décrire divers exemples de réalisation de la présente invention, en référence aux dessins annexés où les mêmes références désignent d'une figure à l'autre des éléments identiques ou fonctionnellement semblables.
La Figure 1 illustre des dispositifs médicaux de type capsule *leadless* dans leur environnement, avec divers exemples de sites d'implantation dans, sur ou à proximité du cœur d'un patient.
La Figure 2 est une coupe longitudinale en vue perspective d'un exemple de capsule *leadless*, montrant la configuration mécanique des différents éléments situés à l'intérieur de l'enveloppe tubulaire de la capsule.
La Figure 3 est une vue perspective générale de l'ensemble frontal selon l'invention, situé à l'extrémité proximale d'une capsule telle que celle de la Figure 2.
La Figure 4 est une vue en coupe, en perspective, de l'ensemble frontal de la Figure 3.
La Figure 5 est une vue éclatée, en perspective, des différents éléments constituant l'ensemble de la Figure 4.
La Figure 6 est une vue en élévation, dans une configuration assemblée, de l'ensemble frontal des Figures 4 et 5 explicitant les deux sens de rotation possibles des éléments mobiles.
La Figure 7 illustre isolément, en élévation et en perspective vue de l'avant, du capot de fermeture solidaire du corps tubulaire.
La Figure 8 illustre isolément, en élévation et en perspective vue de l'arrière, la bague support solidaire de la vis hélicoïdale.
La Figure 9 est une vue en coupe par un plan axial de la bague support de vis de la Figure 8.
La Figure 10 est homologue de la Figure 9, pour une variante de réalisation.

### DESCRIPTION DÉTAILLÉE D'UN MODE DE RÉALISATION

### PRÉFÉRENTIEL DE L'INVENTION

On va maintenant décrire un exemple de réalisation d'une capsule selon l'invention.

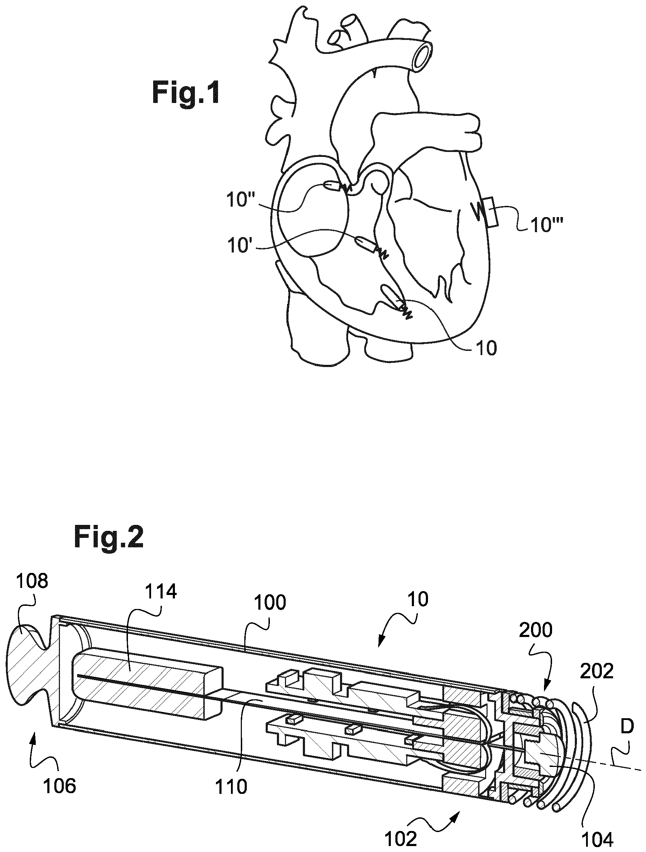

Sur la Figure 1, on a représenté diverses possibilités de sites d'implantation d'un dispositif de type *leadless*, dans une application à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme en 10". Le dispositif peut également être une capsule épicardique placée sur une région externe du myocarde, comme en 10"'. Dans chaque cas, la capsule *leadless* est fixée à la paroi cardiaque au moyen d'un système d'ancrage saillant tel qu'une vis hélicoïdale pénétrant dans le tissu cardiaque pour le maintien sur le site d'implantation.

La Figure 2 est une coupe longitudinale en vue perspective d'un exemple de capsule *leadless* montrant la configuration mécanique des différents éléments situés à l'intérieur de l'enveloppe tubulaire de la capsule.

La capsule *leadless* 10 se présente sous forme extérieure d'un implant comprenant un corps tubulaire allongé cylindrique 100 enfermant les divers circuits électroniques et d'alimentation de la capsule ainsi que, dans l'exemple (non limitatif) illustré, un récupérateur d'énergie à ensemble pendulaire. Les dimensions typiques d'une telle capsule sont un diamètre de l'ordre de 6 mm pour une longueur d'environ 25 à 40 mm.

Le corps tubulaire allongé 100 est fermé à son extrémité avant (distale) 102 par un ensemble frontal 200 portant une vis hélicoïdale 202 pour l'ancrage de la capsule sur une paroi d'une cavité cardiaque, comme illustré plus haut à propos de la Figure 1. Une électrode de détection/ stimulation 104, en contact avec le tissu cardiaque au site d'implantation, assure le recueil des potentiels de dépolarisation cardiaque et/ou l'application d'impulsions de stimulation.

L'extrémité arrière (proximale) opposée 106 du corps tubulaire 100 de la capsule 10 présente une forme arrondie atraumatique, et elle est pourvue de moyens appropriés 108 tels qu'une forme de préhension pour la liaison à un cathéter-guide ou autre accessoire d'implantation utilisable au moment de la mise en place ou de l'explantation de la capsule. Ces moyens permettent, par une action contrôlée par le praticien de rotation et de translation combinées du cathéter-guide, de guider la capsule vers le site d'implantation puis d'y assujettir celle-ci par vissage de l'élément d'ancrage 202 dans la paroi.

Lors de l'explantation, la manœuvre est inverse : le praticien exerce au moyen du cathéter-guide un couple de dévissage sur la forme de préhension en partie proximale de la capsule, couple qui est transmis à l'ensemble frontal 200 et à la vis hélicoïdale 202 pour permettre le dévissage de l'ensemble et la séparation de la capsule d'avec la paroi cardiaque.

La capsule 10 est avantageusement pourvue d'un module de récupération d'énergie comprenant un ensemble pendulaire inertiel qui, à l'intérieur de la capsule, oscille au gré des diverses sollicitations externes auxquelles la capsule est soumise (mouvements de la paroi à laquelle est ancrée la capsule, variations du débit du flux sanguin dans le milieu environnant l'implant, qui produisent des oscillations de l'implant au rythme des battements cardiaques, et/ou vibrations diverses transmises par les tissus cardiaques). Cet ensemble pendulaire peut notamment être de type masse-ressort, avec une lame piézoélectrique 110 encastrée à l'une de ses extrémités et dont l'extrémité opposée, libre, est couplée à une masse inertielle mobile 114.

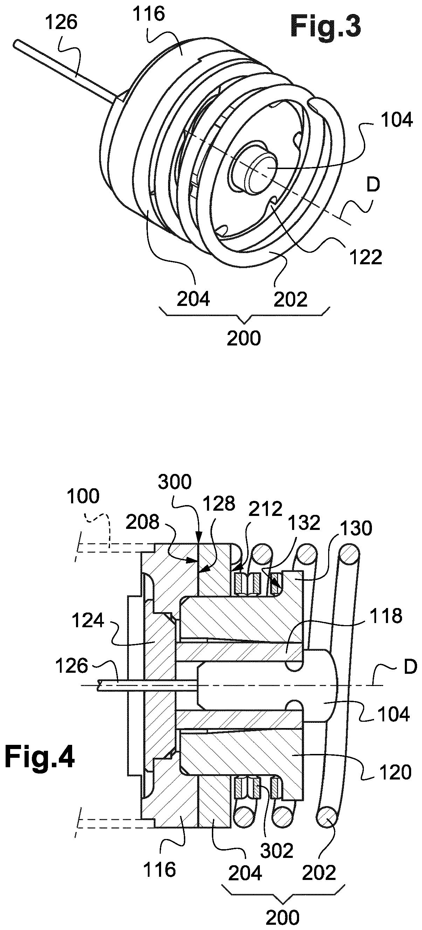

Les Figures 3 et 4 illustrent, isolément, l'ensemble frontal 200 de la capsule monté à l'extrémité distale du corps tubulaire 100 de la capsule, avec l'électrode 104 destinée à venir en appui contre la surface du tissu à l'endroit du site d'implantation et la vis hélicoïdale 202 destinée à pénétrer dans ce tissu pour permettre l'ancrage de la capsule au site d'implantation.

La vis 202 est solidaire en rotation d'une bague support 204, la vis étant par exemple soudée à cette bague support, les deux éléments étant métalliques, par exemple en acier inoxydable tel qu'un acier 316 L ou encore un métal tel que le titane, le tantale ou un alliage nickel-titane de type nitinol.

(On notera incidemment que tous les éléments solidaires du corps tubulaire 100 seront désignés par une référence numérique de type 1 *nn*, tandis que tous ceux qui sont solidaires de l'ensemble frontal 200 seront désignés par une référence numérique de type 2nn).

Le corps tubulaire 100 est terminé en vis-à-vis de la bague support 204 par un capot de fermeture 116 de forme sensiblement plate et contre lequel la bague support 204 vient en appui. Le capot de fermeture 116, tout comme le corps tubulaire 100 de la capsule, sont des éléments métalliques, par exemple en titane ou en acier inoxydable tel qu'un acier 316 L, ou encore en un alliage nickel-titane de type nitinol, les deux pièces 100 et 116 étant par exemple soudées ensemble.

L'ensemble frontal 200, comprenant donc la vis d'ancrage 202 avec sa bague support 204, dispose par rapport au corps tubulaire 100 et à son capot de fermeture 116 d'un degré de liberté en rotation axiale (c'est-à-dire autour de l'axe central D).

Le corps tubulaire 100 ainsi que les éléments dont il est solidaire, d'une part, et l'ensemble frontal 200, d'autre part, sont couplés entre eux par des moyens débrayables à friction, qui peuvent notamment être d'un type décrit dans le US 2020/094048 A1 précité. Ces moyens débrayables sont agencés pour assurer une fonction de limiteur de couple entre le corps tubulaire (qui reçoit une sollicitation externe au moment de l'implantation via l'outil de pose manipulé par le praticien) et la vis d'ancrage (qui pénètre dans le tissu, et pour lequel il est indispensable d'éviter tout effet de carottage).

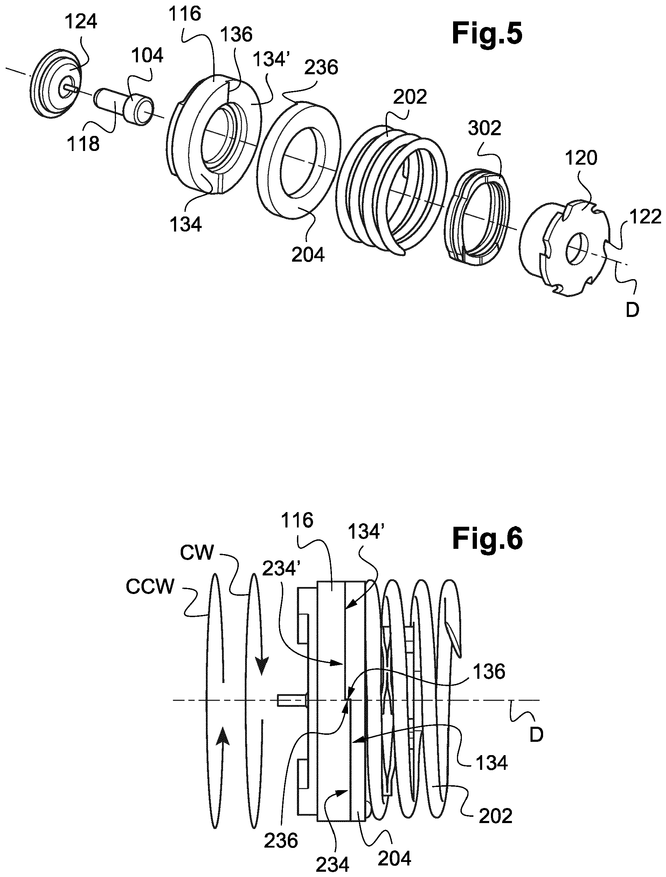

Le mécanisme limiteur de couple permet, lorsque le corps tubulaire est sollicité en rotation dans le sens du vissage (sens CW des aiguilles d'une montre, cf. Figure 6) et applique donc à la vis d'ancrage un couple correspondant qui permet la pénétration de celle-ci dans le tissu, d'assurer la transmission de ce couple d'entrainement tant qu'il est inférieur à un couple seuil prédéterminé (choisi pour être inférieur au couple de carottage) et, si le couple d'entrainement dépasse ce seuil, empêche le carottage, soit en limitant ce couple (qui continuera à être transmis du corps tubulaire à l'ensemble frontal), soit en débrayant totalement l'ensemble frontal du corps tubulaire (mettant ainsi fin à la transmission du couple d'entrainement). Le couple seuil prédéterminé déclenchant le découplage de l'ensemble frontal d'avec le corps tubulaire est typiquement inférieur à 1N.cm, pour éviter tout risque de carottage par la vis hélicoïdale au moment de l'implantation. Concrètement, le couple de réaction augmente de façon relativement brusque lorsque la face avant de la capsule (en l'occurrence, l'électrode 104) touche la surface du tissu cardiaque, exerçant alors sur la vis d'ancrage une force de réaction axiale qui, en l'absence d'action de débrayage ou de limitation du couple, serait susceptible de produire un carottage.

Si l'on souhaite explanter la capsule, un couple de rotation exercé en sens inverse (sens CCW contraire des aiguilles d'une montre, cf. Figure 6) sur le corps tubulaire permettra de transmettre le mouvement de dévissage à la vis hélicoïdale et donc de détacher progressivement la capsule de la paroi.

Le corps tubulaire 100 et son capot de fermeture 116 forment avec l'électrode 104 un ensemble solidaire portée par un manchon support d'électrode 118 en matériau électriquement isolant, par exemple un polymère thermoplastique de polyuréthane de type *Tecothane*^{®} ou un polymère de type PET (poly(téréphtalate d'éthylène)) ou PEEK (polyétheréthercétone), ou autre matière plastique injectable. L'électrode 104 est reliée aux circuits électroniques situés à l'intérieur du corps tubulaire 100 par un conducteur 126 traversant le capot 116 et isolé de celui-ci par une traversée 124.

Le manchon isolant 118 est logé au centre d'une couronne 120 en matériau métallique, soudée côté proximal au capot de fermeture 1.16. Comme on peut le voir sur les Figures 3 et 5, cette couronne 120 peut être avantageusement munie de dents saillantes 122 orientées dans une direction circonférentielle opposée au sens de vissage et renforçant le maintien de la capsule au tissu cardiaque après la pose. Il s'agit de faire en sorte que la capsule ne puisse être séparée de la paroi cardiaque que par l'intervention volontaire d'un praticien et suivant une procédure prédéterminée, mais en aucun cas du fait d'un dévissage accidentel résultant par exemple des mouvements répétés du myocarde ou de la modification des tissus du site d'implantation au fil du temps. Les dents 122 forment autant de points d'ancrage ponctuels répartis sur l'endothélium, rendant la capsule indémontable en l'absence de manœuvre externe d'explantation.

En ce qui concerne l'ensemble frontal 200, qui est mobile en rotation par rapport aux divers éléments 100 à 122 que l'on vient de décrire, cet ensemble comprend la bague support 204 solidaire de la vis hélicoïdale 202 et qui comprend côté proximal une surface d'appui 208 tournée vers une surface homologue 128 du capot de fermeture 116, avec laquelle elle est en contact. Ces deux surfaces 128 et 208, toutes deux métalliques, forment une interface 300 avec un effet de friction mutuel.

Côté distal, la surface 212 de la bague support 204 vient en appui contre un ressort annulaire 302 venant en appui, côté distal, contre une surface 132 formée sur un épaulement 130 de la couronne 120, cette surface 132 s'étendant sensiblement dans un plan radial et étant tournée en direction proximale. Le ressort 302 se trouve ainsi axialement emprisonné entre la bague support 204 (surface 212) et la couronne 120 (surface 132). Ce ressort 302 exerce de ce fait sur la bague support 204 un effort axial en direction du corps tubulaire 100, effort se traduisant par un effet de frottement à l'interface 300 entre le capot de fermeture 116 (surface 128) et la bague support 204 (surface 208).

Ce mécanisme de couplage frottant entre le capot de fermeture 116, et donc le corps tubulaire 100, d'une part, et la bague support 204 et donc la vis hélicoïdale 202, d'autre part, permet au moment de l'implantation de limiter le couple transmis par le corps tubulaire 100 (entraîné dans le sens de vissage CW, qui est le sens des aiguilles d'une montre sur la Figure 6) à la vis d'ancrage 202 et d'obtenir ainsi l'effet limiteur de couple anticarottage recherché.

On notera également qu'après avoir ancré la capsule dans la paroi, il reste toujours possible de réorienter axialement le corps tubulaire sans exercer d'action de rotation sur la vis, donc sans risque de survissage pouvant entraîner un carottage. Cette faculté peut se révéler intéressante dans le cas d'une capsule munie d'un récupérateur d'énergie tel que celui illustré Figure 2, avec un système pendulaire à masse inertielle et lame flexible ; avec cette configuration, la récupération d'énergie peut varier en fonction de l'orientation dans l'espace de la direction de flexion dans la lame par rapport à la paroi cardiaque, de sorte qu'il peut être avantageux d'optimiser la conversion d'énergie en faisant tourner le corps de la capsule jusqu'à trouver la position qui maximise cette conversion.

Pour le ressort 300, il est avantageux de choisir un ressort ondulé, qui présente l'avantage d'une très grande compacité, compatible avec les exigences de miniaturisation extrême des capsules leadless. L'avantage d'un tel ressort ondulé est le gain de place axial qu'il permet, typiquement de 50 % par rapport à un ressort filaire hélicoïdal conventionnel. D'autres types de ressorts ou éléments fonctionnellement analogues sont toutefois utilisables tels que : entretoise en matériau souple, par exemple en silicone, ressort hélicoïdal de compression, lame-ressort, bague plastique déformable, etc., l'important étant que cet élément puisse engendrer entre les surfaces 208 et 128 à l'interface 300 un frottement suffisant pour limiter le couple à la valeur prédéterminée choisie, tout en maintenant l'orientation angulaire du corps tubulaire 100 par rapport à l'ensemble frontal 200 en l'absence de couple appliqué de l'extérieur au corps tubulaire 100.

De façon caractéristique de la présente invention, les surfaces conjuguées 208 (de la bague support 204) et 128 (du capot de fermeture 116) comportent un mécanisme opérant au cours d'une opération d'explantation, en plus du mécanisme à friction que l'on vient de décrire et qui permet d'obtenir un effet anticarottage à l'implantation.

Ce mécanisme selon l'invention intervient lorsque le corps tubulaire est soumis par un praticien, au moyen d'un cathéter couplé au corps tubulaire, à une rotation dans le sens contraire des aiguilles d'une montre (sens CCW sur la Figure 6) pour dévisser la vis hélicoïdale 202 afin de détacher la capsule du site d'implantation auquel elle est ancrée.

Avec le seul mécanisme à friction limiteur de couple que l'on vient de décrire, si l'on exerce un couple de dévissage supérieur au couple limite autorisé à l'interface 300 (couple limite défini par la pression du ressort 302), le corps tubulaire 100 continue à tourner mais sans que cette rotation soit transmise à la vis hélicoïdale 202. Cette situation peut notamment se présenter lorsqu'une fibrose s'est développée au fil du temps à l'endroit du site d'implantation, fibrose qui accroit fortement l'adhérence de la vis au tissu cardiaque et augmente de ce fait le couple nécessaire pour dévisser de la vis hélicoïdale 202. Par ailleurs, la présence éventuelle sur la couronne 120 de dents d'accrochage 122 augmente l'effort qu'il est nécessaire d'exercer au dévissage, dans la mesure où les arêtes vives de ces dents auront tendance à pénétrer dans les tissus lors du dévissage.

Pour pallier ce risque et empêcher tout débrayage du mécanisme de couplage lors d'une manœuvre de dévissage, il est prévu selon l'invention un mécanisme de blocage de la rotation mutuelle du capot de fermeture 116 et de la bague support 204, ce mécanisme étant un mécanisme unidirectionnel, interdisant le débrayage dans le sens du dévissage mais étant sans effet dans le sens du vissage (c'est-à-dire que dans le sens du vissage les deux pièces homologues 116 et 124 sont soumises à un contact frottant, calibré par la pression du ressort 302, donc avec possibilité de rotation mutuelle lorsque le couple de réaction de la vis hélicoïdale 202 dépasse le seuil prédéterminé évitant tout carottage).

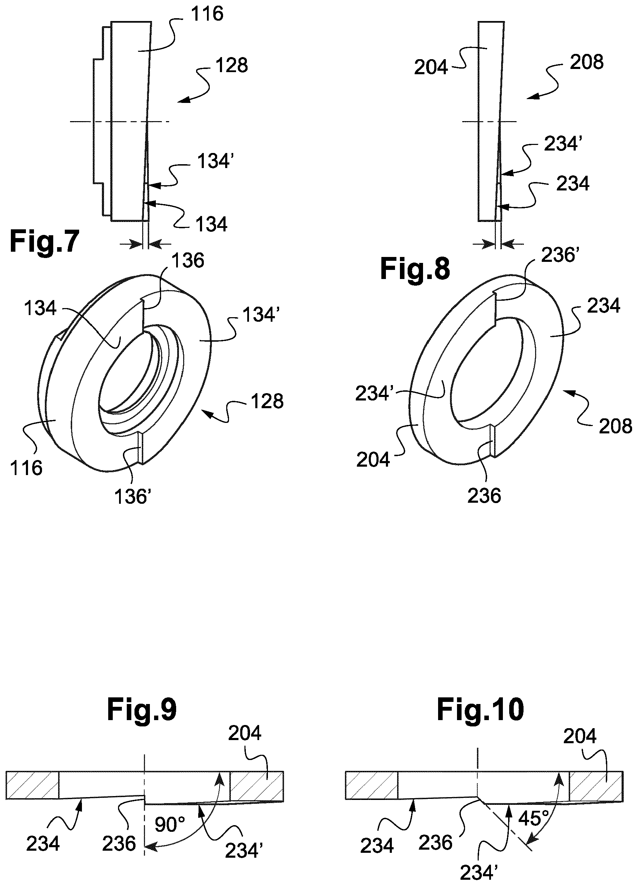

Dans le mode de réalisation illustré Figures 5 à 10, ce mécanisme unidirectionnel est réalisé en donnant à la surface 128 du capot de fermeture la forme d'un plateau annulaire avec une pluralité de secteurs circulaires, dans l'exemple illustré deux secteurs circulaires annulaires 134, 134', légèrement décalés en sens inverses l'un par rapport à l'autre par rapport à un plan radial de référence, de manière à former au niveau du raccordement entre ces deux secteurs circulaires 134, 134' deux gradins 136, 136' (voir notamment Figure 7) orientés radialement.

De façon comparable, la surface 208 en vis-à-vis de la face proximale de la bague support 204 est réalisée sous forme d'un plateau annulaire avec deux secteurs circulaires annulaires 234, 234' légèrement décalés en sens inverses l'un par rapport à l'autre par rapport à un plan radial de référence, de manière à former au niveau du raccordement entre ces deux secteurs circulaires 234, 234' des gradins 236, 236' orientés radialement.

Les gradins 136, 136' et 236, 236' présentent typiquement une hauteur comprise entre 3 et 7 %, par exemple 5 %, du rayon de la surface respective 128, 208, soit une hauteur de gradin d'environ 0,2 mm pour un rayon de 3,8 mm.

Les surfaces des secteurs circulaires annulaires 134, 134' et 234, 234' sont choisies de manière que les deux pièces homologues 116 et 204 restent en contact mutuel l'une avec l'autre (surface 134 contre surface 234 et surface 134' contre surface 234') de manière à maintenir un couplage frottant dans le sens du vissage.

En revanche, dans le sens du dévissage, les gradins 136, 136' du capot de fermeture vont venir en butée contre les gradins homologues 236, 236' de la bague support 204, ce qui aura pour effet de neutraliser le couplage frottant et d'imposer une rotation axiale à la bague support 204, et donc à la vis hélicoïdale 202, sous l'effet de la rotation appliquée au corps tubulaire 100, et donc au capot de fermeture 116 en contact avec la bague support 204.

La configuration de l'exemple illustré, avec deux demi-secteurs circulaires annulaires plans et inclinés n'est toutefois pas limitative de l'invention. Le nombre des secteurs pourrait être différent (un, trois, quatre, ...), et les secteurs non nécessairement plans, dès lors qu'au moins un gradin radial peut être formé sur au moins l'une des deux pièces 116 ou 204, avec sur la pièce conjuguée en vis-à-vis 204 ou 116 une configuration de forme permettant de venir en butée avec ce(s) gradin(s), qu'il s'agisse d'un gradin semblable à celui de la première pièce comme dans l'exemple illustré, ou d'une autre forme de butée telle que bossage, pion ou épaulement, dès lors que l'effet de mécanisme antiretour est obtenu dans le sens du dévissage tout en continuant à assurer le couplage frottant dans le sens du vissage.

Selon une autre caractéristique, subsidiaire et avantageuse, de l'invention, l'angle du gradin 136, 236, ou de la formation de butée équivalente, peut être modifié pour assurer une fonction supplémentaire de limitation de couple, au dévissage.

En effet, dans la description ci-dessus, il a toujours été considéré que le mécanisme anti-débrayage unidirectionnel opérait quel que soit le couple de dévissage appliqué au corps tubulaire 100.

Il peut être toutefois souhaitable de limiter ce couple à une valeur limite, relativement élevée, en cas de couple de réaction excessif de la vis hélicoïdale du fait d'une adhérence excessive des tissus, notamment du fait d'une fibrose développée au cours du temps. Dans ce cas, il est préférable de ne pas tenter d'explanter la capsule par un couple de dévissage excessif, au risque de déchirer les tissus et d'entraîner une perforation de la paroi cardiaque.

Pour pallier ce risque, au lieu de prévoir pour les gradins 136 et/ou 236, comme dans le cas précédent, un angle de 90 degrés (comme illustré Figure 9) par rapport aux plans des faces correspondantes 134, 134' et/ou 234, 234', cet angle est réduit, par exemple à 45 degrés (comme illustré Figure 10). Avec un tel angle réduit, le dévissage restera possible jusqu'à un certain couple limite, avec une désolidarisation en rotation axiale des deux pièces 116, 204 par saut du gradin en cas de couple de réaction excessif de la vis hélicoïdale (le couple limite étant d'autant plus faible que l'angle d'inclinaison du gradin est éloigné de l'angle droit). Pour le cas évoqué plus haut d'une butée de type bossage ou pion, un effet comparable peut être obtenu en donnant à cette butée une forme inclinée ou arrondie.

Un autre avantage d'un angle inférieur à 90 degrés réside dans une protection contre le risque de détachement de particules lorsque le couple limite de débrayage est atteint et que les deux pièces 116, 204 se désolidarisent en rotation axiale et glissent l'une sur l'autre. En effet, avec un gradin incliné le déplacement relatif de ces deux pièces se fait alors de façon progressive, et non de façon brusque comme avec le saut d'un gradin à 90 degrés.

## Revendications

1. Une capsule autonome implantable, comprenant :
- un corps tubulaire (100) logeant un ensemble d'éléments fonctionnels de la capsule ;
- à une extrémité distale (102) de la capsule, un ensemble frontal (200) comprenant un organe d'ancrage à vis hélicoïdale (202) pour l'ancrage de la capsule à une paroi d'un organe du patient ; et
- un couplage débrayable à friction agencé entre l'ensemble frontal (200) et le corps tubulaire (100),
dans laquelle l'ensemble frontal (200) et la vis hélicoïdale (202) sont mobiles ensemble en rotation relative axiale par rapport au corps tubulaire (100),
et dans laquelle le couplage débrayable à friction est agencé pour, lorsqu'une sollicitation externe en rotation axiale est appliquée au corps tubulaire (100) dans un premier sens (CW) correspondant à un sens de vissage de la vis hélicoïdale (202) :
• empêcher la rotation relative axiale tant qu'un couple de réaction, exercé par la vis hélicoïdale (202) au vissage dans la paroi de l'organe du patient, est inférieur à un premier couple seuil prédéterminé, et
• autoriser la rotation relative axiale dès que le couple de réaction dépasse le premier couple seuil prédéterminé,
***caractérisée en ce que*** le couplage débrayable à friction comporte en outre un mécanisme unidirectionnel de blocage au dévissage,
ce mécanisme unidirectionnel de blocage étant agencé pour, lorsqu'une sollicitation externe en rotation axiale est appliquée au corps tubulaire (100) dans un second sens (CCW), inverse du premier sens (CW) et correspondant à un sens de dévissage de la vis hélicoïdale (202) :
• empêcher le débrayage du mécanisme de couplage, en interdisant la rotation relative axiale à l'encontre d'un couple résistant exercé sur l'ensemble frontal (200) par la vis hélicoïdale (202) au dévissage et permettre ainsi une rotation conjointe du corps tubulaire (100) et de l'ensemble frontal (200) ; et
• être sans effet dans le sens du vissage (CW).

2. La capsule de la revendication 1, dans laquelle le couplage débrayable à friction comprend deux plateaux conjugués (128, 208) en vis-à-vis, avec i) un premier plateau (128) s'étendant radialement et solidaire du corps tubulaire (100) et ii) un second plateau (208) s'étendant radialement et solidaire de l'ensemble frontal (200), et le mécanisme unidirectionnel de blocage est formé par les deux plateaux conjugués (128, 208) qui possèdent des configurations de surface respectives formant mécanisme anti-retour pour, lorsque les deux plateaux sont en contact mutuel, autoriser la rotation relative des deux plateaux dans le premier sens (CW) et empêcher la rotation relative des deux plateaux dans le second sens (CCW).

3. La capsule de la revendication 2, dans laquelle :
- les configurations de surface respectives en vis-à-vis et en contact des deux plateaux conjugués (128, 208) forment une interface de friction (300) ; et
- l'organe de couplage débrayable comprend un élément élastiquement déformable (302) agencé pour appliquer un effort de compression axiale des deux plateaux conjugués (128, 208) l'un contre l'autre.

4. La capsule de la revendication 3, dans laquelle, en l'absence de sollicitation externe en rotation appliquée au corps tubulaire (100), l'élément élastiquement déformable (302) applique à l'endroit de l'interface de friction (300) un effort suffisant pour empêcher la rotation relative axiale tant que le couple de réaction exercé par la vis hélicoïdale (202) au vissage dans la paroi de l'organe du patient reste inférieur au premier couple seuil prédéterminé, et autoriser la rotation relative axiale dès que le couple de réaction dépasse le premier couple seuil prédéterminé.

5. La capsule de la revendication 2, dans laquelle au moins l'un du premier et du second plateau (128 ; 208) comprend une pluralité de secteurs circulaires (134, 134' ; 234, 234') décalés en sens inverses par rapport à un plan radial de référence, avec un gradin (136 ; 236) s'étendant radialement à chaque transition entre secteurs circulaires (134, 134' ; 234, 234') adjacents.

6. La capsule de la revendication 5, dans laquelle les gradins (136 ; 236) sont sensiblement perpendiculaires à la surface des secteurs circulaires (134, 134' ; 234, 234').

7. La capsule de la revendication 5, dans laquelle les gradins (136 ; 236) sont inclinés d'un angle inférieur à 90° par rapport à la surface des secteurs circulaires (134, 134' ; 234, 234'), de préférence inclinés d'un angle de 45°.

8. La capsule de la revendication 5, dans laquelle les secteurs circulaires (134, 134' ; 234, 234') sont des secteurs plans.

9. La capsule de la revendication 5, dans laquelle le premier et le second plateau (128, 208) comprennent tous deux une pluralité de secteurs circulaires (134, 134', 234, 234'), formant respectivement des gradins symétriques (136 ; 236) en vis-à-vis.

10. La capsule de la revendication 5, dans laquelle les gradins (136 ; 236) présentent une hauteur comprise entre 3 et 7 %, de préférence une hauteur de 5 %, du rayon des secteurs circulaires (134, 134' ; 234, 234').

11. La capsule de la revendication 2, dans laquelle le premier et le second plateau (128, 208) comprennent une pluralité de bossages et/ou alvéoles conjugués formant le mécanisme anti-retour.

12. La capsule de la revendication 1, dans laquelle le mécanisme unidirectionnel de blocage est en outre agencé pour, lorsque la sollicitation externe en rotation axiale dans le second sens (CCW) est appliquée au corps tubulaire (100) :
• empêcher la rotation relative axiale tant que le couple résistant exercé sur l'ensemble frontal (200) par la vis hélicoïdale (202) au dévissage reste inférieur à un second couple seuil prédéterminé, et
• autoriser la rotation relative axiale lorsque le couple résistant exercé sur l'ensemble frontal (200) par la vis hélicoïdale (202) au dévissage dépasse le second couple seuil prédéterminé.

## Patentansprüche

1. Implantierbare autonome Kapsel, umfassend:
- einen rohrförmigen Körper (100), der eine Anordnung aus Funktionselementen der Kapsel aufnimmt;
- an einem distalen Ende (102) der Kapsel, eine frontale Anordnung (200), umfassend ein Verankerungsorgan mit einer schraubenförmigen Schnecke (202) für die Verankerung der Kapsel an einer Wand eines Organs eines Patienten; und
- eine auskuppelbare Reibkupplung, die zwischen der frontalen Anordnung (200) und dem rohrförmigen Körper (100) eingerichtet ist,
wobei die frontale Anordnung (200) und die schraubenförmige Schnecke (202) gemeinsam in axialer relativer Drehung relativ zu dem rohrförmigen Körper (100) beweglich sind
und wobei die auskuppelbare Reibkupplung eingerichtet ist, um, wenn eine externe axiale Drehbeaufschlagung auf den rohrförmigen Körper (100) in einer ersten Richtung (CW), die einer Einschraubrichtung der schraubenförmigen Schnecke (202) entspricht, aufgebracht wird:
• die axiale relative Drehung zu verhindern, solange ein Reaktionsmoment, das von der schraubenförmigen Schnecke (202) beim Einschrauben in die Wand des Organs des Patienten ausgeübt wird, kleiner ist als ein erstes vorher bestimmtes Schwellenmoment, und
• die axiale relative Drehung freizugeben, sobald das Reaktionsmoment das erste vorher bestimmte Schwellenmoment übersteigt,
***dadurch gekennzeichnet, dass*** die auskuppelbare Reibkupplung ferner einen unidirektionalen Sperrmechanismus gegenüber dem Ausschrauben aufweist, wobei dieser unidirektionale Sperrmechanismus eingerichtet ist, um, wenn eine externe axiale Drehbeaufschlagung auf den rohrförmigen Körper (100) in einer zweiten Richtung (CCW), die zu der ersten Richtung (CW) entgegengesetzt ist und einer Ausschraubrichtung der schraubenförmigen Schnecke (202) entspricht, aufgebracht wird:
• das Entkuppeln des Kupplungsmechanismus zu verhindern, indem die axiale relative Drehung gegen ein Widerstandsmoment, das beim Ausschrauben von der schraubenförmigen Schnecke (202) auf die frontale Anordnung (200) ausgeübt wird, verhindert wird, und auf diese Weise eine gemeinsame Drehung des rohrförmigen Körpers (100) und der frontalen Anordnung (200) zu gestatten; und
• in der Einschraubrichtung (CW) wirkungslos zu sein.

2. Kapsel nach Anspruch 1, wobei die auskuppelbare Reibkupplung zwei angepasste Platten (128, 208) umfasst, die gegenüberliegend angeordnet sind, mit i) einer ersten Platte (128), die sich radial und mit dem rohrförmigen Körper (100) fest verbunden erstreckt Und II) sich eine zweite Platte (208) radial und mit der frontalen Anordnung (200) fest verbunden erstreckt, und der unidirektionale Sperrmechanismus von den zwei angepassten Platten (128, 208) gebildet ist, die jeweilige Oberflächengestaltungen aufweisen, die einen Rückschlagmechanismus bilden, um, wenn die zwei Platten in gegenseitigem Kontakt sind, die relative Drehung der zwei Platten in der ersten Richtung (CW) freizugeben und die relative Drehung der zwei Platten in der zweiten Richtung (CCW) zu verhindern.

3. Kapsel nach Anspruch 2, wobei:
- die gegenüberliegend und in Kontakt angeordneten jeweiligen Oberflächengestaltungen der zwei angepassten Platten (128, 208) eine Reibungsschnittstelle (300) bilden; und
- das auskuppelbare Kupplungsorgan ein elastisch verformbares Element (302) umfasst, das eingerichtet ist, um eine axiale Druckkraft der zwei angepassten Platten (128, 208) gegeneinander aufzubringen.

4. Kapsel nach Anspruch 3, wobei, bei Fehlen einer auf den rohrförmigen Körper (100) aufgebrachten externen Drehbeaufschlagung, das elastisch verformbare Element (302) auf die Stelle der Reibungsschnittstelle (300) eine Kraft aufbringt, die ausreichend ist, um die axiale relative Drehung zu verhindern, solange das Reaktionsmoment, das von der schraubenförmigen Schnecke (202) beim Einschrauben in die Wand des Organs des Patienten ausgeübt wird, kleiner bleibt das erste vorher bestimmte Schwellenmoment, um die axiale relative Drehung freizugeben, sobald das Reaktionsmoment das erste vorher bestimmte Schwellenmoment übersteigt.

5. Kapsel nach Anspruch 2, wobei wenigstens eine von der ersten und der zweiten Platte (128; 208) eine Mehrzahl kreisförmiger Sektoren (134, 134'; 234, 234') umfasst, die in entgegengesetzten Richtungen relativ zu einer radialen Referenzebene versetzt sind, mit einer Stufe (136; 236), die sich radial zu jedem Übergang zwischen benachbarten kreisförmigen Sektoren (134, 134'; 234, 234') erstreckt.

6. Kapsel nach Anspruch 5, wobei die Stufen (136; 236) zu der Oberfläche der kreisförmigen Sektoren (134, 134'; 234, 234') im Wesentlichen senkrecht sind.

7. Kapsel nach Anspruch 5, wobei die Stufen (136; 236) in einem Winkel kleiner 90° relativ zu der Oberfläche der kreisförmigen Sektoren (134, 134'; 234, 234') geneigt sind, bevorzugt in einem Winkel kleiner 45° geneigt sind.

8. Kapsel nach Anspruch 5, wobei die kreisförmigen Sektoren (134, 134'; 234, 234') flächige Sektoren sind.

9. Kapsel nach Anspruch 5, wobei die erste und die zweite Platte (128, 208) beide eine Mehrzahl kreisförmiger Sektoren (134, 134', 234, 234') umfassen, die jeweils gegenüberliegend angeordnete symmetrische Stufen (136; 236) bilden.

10. Kapsel nach Anspruch 5, wobei die Stufen (136; 236) eine Höhe zwischen 3 und 7 %, bevorzugt eine Höhe von 5 %, des Radius der kreisförmigen Sektoren (134, 134'; 234, 234') aufweisen.

11. Kapsel nach Anspruch 2, wobei die erste und die zweite Platte (128, 208) eine Mehrzahl von angepassten Höckern und/oder Waben umfassen, die den Rückschlagmechanismus bilden.

12. Kapsel nach Anspruch 1, wobei der unidirektionale Sperrmechanismus ferner eingerichtet ist, um, wenn die externe axiale Drehbeaufschlagung in der zweiten Richtung (CCW) auf den rohrförmigen Körper (100) aufgebracht wird:
• die axiale relative Drehung zu verhindern, solange das Widerstandsmoment, das auf beim Ausschrauben von der schraubenförmigen Schnecke (202) auf die frontale Anordnung (200) ausgeübt wird, kleiner bleibt ein zweites vorher bestimmtes Schwellenmoment, und
• die axiale relative Drehung freizugeben, wenn das Widerstandsmoment, das beim Ausschrauben von der schraubenförmigen Schnecke (202) auf die frontale Anordnung (200) ausgeübt wird, das zweite vorher bestimmte Schwellenmoment überschreitet.

## Claims

1. An implantable autonomous capsule, comprising:
- a tubular body (100) housing a set of functional components of the capsule;
- at a front, distal end (102) of the capsule, a front-end unit (200) comprising a helical-screw anchoring member (202) for anchoring the capsule to a wall of a patient's organ; and
- a disengageable frictional coupling, arranged at said distal end of the capsule between the front-end unit (200) and the tubular body (100),
wherein the front-end unit (200) and the helical screw (202) are mobile as a whole in relative axial rotation with respect to the tubular body (100), and wherein the disengageable frictional coupling is arranged in such a way as, when an external axial rotational stress is applied to the tubular body (100) at the proximal end in a first direction (CW) corresponding to a screwing direction of the helical screw (202):
• to prevent the relative axial rotation as long as a reaction torque, exerted by the helical screw (202) during the screwing into the wall of the patient's organ, is lower than a first predetermined threshold torque, and
• to allow the relative axial rotation as soon as the reaction torque exceeds the first predetermined threshold torque,
***characterized in that*** the disengageable frictional coupling further includes a one-way unscrewing blocking mechanism,
said one-way blocking mechanism being arranged in such a way as, when an external axial rotational stress is applied to the tubular body (100) at the proximal end in a second direction (CCW), opposite to the first direction (CW) and corresponding to an unscrewing direction of the helical screw (202):
• to prevent a disengagement of the coupling mechanism, by forbidding the relative axial rotation against a resistive torque exerted on the front-end unit (200) by the helical screw (202) during an unscrewing, whereby allowing a joint rotation of the tubular body (100) and the front-end unit (200); and
• to be without effect in the screwing direction (CW).

2. The capsule of claim 1, wherein the disengageable frictional coupling comprises two conjugated plates (128, 208) facing each other, with i) a first plate (128) extending radially and integral with the tubular body (100) and ii) a second plate (208) extending radially and integral with the front-end unit (200), and the one-way blocking mechanism is formed by the two conjugated plates (128, 208) that have respective surface configurations forming an anti-return mechanism in such a was as, when the two plates are in mutual contact, to allow the relative rotation of the two plates in the first direction (CW) and to prevent the relative rotation of the two plates in the second direction (CCW).

3. The capsule of claim 2, wherein:
- the respective surface configurations facing each other and in contact with the two conjugated plates (128, 208) form a friction interface (300); and
- the disengageable coupling member comprises an elastically deformable element (302) adapted to apply an axial force of compression of the two conjugated plates (128, 208) against each other.

4. The capsule of claim 3, wherein, in the absence of an external rotational stress applied to the tubular body (100), the elastically deformable element (302) applies at the friction interface (300) a sufficient force to prevent the relative axial rotation as long as the reaction torque exerted by the helical screw (202) during the screwing into the wall of the patient's organ remains lower than the first predetermined threshold torque, and to allow the relative axial rotation as soon as the reaction torque exceeds the first predetermined threshold torque.

5. The capsule of claim 2, wherein at least one of the first and second plates (128, 208) comprises a plurality of circular sectors (134, 134'; 234, 234') offset in opposite directions with respect to a radial reference plane, with a step (136; 236') extending radially at each transition between adjacent circular sectors (134, 134' ; 234, 234').

6. The capsule of claim 5, wherein the steps (136 ; 236') are substantially perpendicular to the surface of the circular sectors (134, 134' ; 234, 234').

7. The capsule of claim 5, wherein the steps (136 ; 236') are inclined by an angle lower than 90° with respect to the surface of the circular sectors (134, 134' ; 234, 234'), preferably inclined by an angle of 45°.

8. The capsule of claim 5, wherein the circular sectors (134, 134' ; 234, 234') are planar sectors.

9. The capsule of claim 5, wherein the first and the second plates (128, 208) both comprise a plurality of circular sectors (134, 134', 234, 234') respectively forming symmetrical steps (136; 236) facing each other.

10. The capsule of claim 5, wherein the steps (136 ; 236) have a height between 3 and 7 %, preferably a height of 5 %, of the radius of the circular sectors (134, 134' ; 234, 234').

11. The capsule of claim 2, wherein the first and the second plates (128, 208) comprise a plurality of conjugated bosses and/or recesses forming the anti-return mechanism.

12. The capsule of claim 1, wherein the one-way blocking mechanism is further arranged in such a way as, when the external axial rotational stress in the second direction (CCW) is applied to the tubular body (100):
- to prevent the relative axial rotation as long as the reaction torque exerted on the front-end unit (200) by the helical screw (202) during the unscrewing remains lower than a second predetermined threshold torque, and
- to allow the relative axial rotation when the resistive torque exerted on the front-end unit (200) by the helical screw (202) during the unscrewing exceeds the second predetermined threshold torque.
